# EUROPEAN PATENT SPECIFICATION

(11) **EP 3 970 549 B1**
(45) Date of publication and mention of the grant of the patent: **03.09.2025**
(21) Application number: 20883547.0
(22) Date of filing: 29.10.2020
(51) Int. Cl.: A43B 17/00, A43B 17/14, A61F 5/14, A43B 7/142, A43B 7/143

(54) **SHOE INSOLE**
SCHUHINNENSOHLE
SEMELLE INTÉRIEURE DE CHAUSSURE

(30) Priority: 30.10.2019 JP 2019197982
(43) Date of publication of application: 23.03.2022
(73) Proprietor: Winning One Co., Ltd., Setagaya-ku Tokyo 157-0062 (JP)
(72) Inventor: HIROSE, Hayato, Tokyo 157-0062 (JP)
(74) Representative: Dolleymores
(86) International application number: PCT/JP2020/040727
(87) International publication number: WO 2021/085574

(56) References cited:
- EP-A2- 0 173 396
- JP-A- 2005 160 727
- JP-A- 2010 125 100
- JP-A- 2011 056 296
- JP-A- 2015 085 134
- JP-A- H0 779 805
- JP-A- S61 168 301
- JP-U- 3 167 048
- JP-U- H0 531 606
- US-A1- 2015 121 722

## Description

### TECHNICAL FIELD

The present invention relates to a shoe insole.

### BACKGROUND ART

During exercise, a person may experience pain and distortion in their body due to the burden on the body, especially the joints of the foot and in turn the legs and back. In consideration of such pain and distortion, the inventor of the present application has been developing, as an invention similar to that of the present application, a shoe insole with which the balance of the skeletal structure of the foot can be regulated when the shoe is worn (for example, see Patent Document 1 below).

In the shoe insole disclosed in Patent Document 1, a center convex section and a center concave section on both sides of the center convex section are provided on an upper surface side of a sheet section, on which approximately the entire sole of the foot is placed, so as to oppose a center portion of the sole of the foot slightly more toward the heel than the ball of the foot. Further, the shoe insole disclosed in Patent Document 1 comprises a hard support plate which is attached so that an area from approximately the center to a portion corresponding to the heel on the bottom surface side of the sheet section can be substantially covered. In the support plate, two rows of through-holes into which portions of the sheet section are fitted are formed approximately along the edges on both sides of the support plate. Further, in the support plate, a ridge section extending in the longitudinal direction of the insole is formed on the inside of the two rows of through-holes.

Due to the above configuration, the insole of Patent Document 1 achieves an extremely superior effect in which the skeletal structure of the foot can be arranged at a preferred position, and the burden caused by distortion on the foot and body when the user is exercising, such as walking and the like, can be reduced.

### PRIOR ART DOCUMENTS

### PATENT DOCUMENTS

Patent Document 1: JP2015-085134A

Other prior art arrangements of shoe insoles are known from US 2015/121722 A1 and EP 0173396 A2.

### SUMMARY OF INVENTION

### TECHNICAL PROBLEM

The feet are particularly constituted by a combination of many bones, and the feet support the weight of the entire body, and thus a person's posture can become unbalanced with even just a slight deviation of the foot joints. Further, deviations of the foot joints can also cause daily burdens on completely different areas besides the feet because the foot joints also attempt to compensate for loss of balance in joints above the feet as well, and this can lead to bodily injuries. Therefore, there is a need to naturally maintain appropriate meshing of the joints at a higher level without needlessly restricting the movement of the foot.

Thus, the inventor has developed a shoe insole with which appropriate meshing of the joints can be naturally maintained at a higher level without needlessly restricting the movement of the foot.

### SOLUTION TO PROBLEM

According to the present invention, there is provided a shoe insole as claimed in claim 1. Preferred but optional features are set out in the dependent claims. A shoe insole of the present invention comprises: a sheet section disposed on the entire base of a shoe, the sheet section including a toe section formed such that the toes of the foot can be disposed therein, a bulged section formed such that the tips of the metatarsal bones at which the width of the foot protrudes farthest to the left and right can be disposed therein, an arch section formed such that a section of the foot from the metatarsal bones to directly below the lateral malleolus can be disposed therein, and a heel section formed such that a section of the foot from directly below the lateral malleolus to the heel bone rearward of the lateral malleolus can be disposed; and a support plate that is adhered to a bottom surface of the arch section and the heel section of the sheet section, and that is formed from a material which is harder than the sheet section. The sheet section and the support plate each comprises in the arch section: an inner wall section curved in an arch shape with an end section standing upward such that at least a first metatarsal bone is disposed therein; an outer wall section curved in an arch shape with an end section standing upward such that at least a fifth metatarsal bone is disposed therein; and a central wall section formed between the inner wall section and the outer wall section. The support plate comprises an inner ridge section formed on a boundary between the inner wall section and the central wall section, and an outer ridge section formed on approximately a boundary between the outer wall section and the central wall section. A plurality of through-holes, arranged in the longitudinal direction of the support plate, are formed in the inner wall section and the outer wall section of the support plate. At least an upper surface of the central wall section located between the inner ridge section and the outer ridge section is curved so as to draw an arch in the longitudinal direction of the support plate.

According to the above configuration, the arch shape of a central rear wall section of the sheet section is supported by the upper surface shape of a central rear wall section of the support plate, and thus an arch shape is properly formed in the foot when the foot is placed on the upper surface of the sheet section.

The sheet section and the support plate of the shoe insole of the present invention may include an extension section that protrudes upward so as to oppose, from a side surface, the heel section located directly below the lateral malleolus and/or a base end section of the arch section.

According to the above configuration, the heel section located directly below the lateral malleolus and/or the base end section of the arch section, i.e. the lower portion of the cuboid bone, is supported from the outside, and thus collapse of the skeletal structure of the foot can be prevented.

The support plate of the shoe insole of the present invention may be formed with a degree of hardness of from 40D to less than 72D, and may be arranged along a bottom surface shape of the sheet section with a fixed thickness.

According to the above configuration, the burden on the foot caused by the support plate can be reduced.

The support plate of the shoe insole of the present invention may be formed with a degree of hardness of from 40D to less than 65D, and may be arranged along a bottom surface shape of the sheet section with a fixed thickness.

According to the above configuration, the burden on the foot caused by the support plate can be further reduced.

In a heel section of the support plate of the shoe insole of the present invention, an opening into which a portion of the sheet section is fitted may be formed.

According to the above configuration, the body weight, which tends to be put on the heel section due to a longitudinal arch shape which is formed in the foot by an arch shape formed by the central rear wall section of the sheet section and the support plate, can be appropriately received at the heel section, and the balance of the body weight on the feet becomes easier to maintain.

The sheet section of the shoe insole of the present invention preferably comprises at least two foamed resin layers having different degrees of hardness and impact resilience coefficients, wherein a first layer on an upper surface side and a second layer provided below the first layer constitute impact resilient resin layers comprising, as a main component, an ethylene-vinyl acetate copolymer.

Further, the measured degrees of hardness as measured using an Asker rubber durometer C-type and the impact resilience coefficients as measured according to ISO4662 of the first layer and the second layer preferably satisfy the following relationships.
Measured Degree of Hardness: the first layer < the second layer
Impact Resilience Coefficient: the first layer < the second layer

More specifically, the measured degree of hardness as measured using an Asker rubber durometer C-type of the second layer is preferably 50 to 60, and the impact resilience coefficient as measured according to ISO4662 of the second layer is preferably 55% to 65%. Further, the measured degree of hardness as measured using an Asker rubber durometer C-type of the first layer is preferably 23 to 28, and the impact resilience coefficient as measured according to ISO4662 of the first layer is preferably 47% to 53%.

### EFFECTS OF INVENTION

The present invention achieves an effect of enabling the appropriate meshing of the joints to be naturally maintained at a higher level without needlessly restricting the movement of the foot.

### BRIEF DESCRIPTION OF DRAWINGS

FIG. 1 is a bottom surface view illustrating a shoe insole according to one embodiment of the present invention.
FIG. 2 is a right-side surface view illustrating the shoe insole according to one embodiment of the present invention.
FIG. 3(a) is a schematic view illustrating the skeletal structure of the right foot when viewed from the front surface, and FIG. 3(b) is a schematic view illustrating the skeletal structure of the right foot when viewed from the outside (little toe side).
FIG. 4 is a cross-section view along the arrowed line A-A shown in FIG. 1.
FIG. 5 is an upper surface view illustrating the shoe insole according to one embodiment of the present invention.
FIG. 6(a) is a bottom surface view illustrating a support plate of the shoe insole according to one embodiment of the present invention, and FIG. 6(b) is a cross-section view along the arrowed line B-B.
FIG. 7 is a left-side surface view illustrating the shoe insole according to one embodiment of the present invention.

### DESCRIPTION OF EMBODIMENTS

In the following, embodiments of the shoe insole of the present invention shall be explained while referring to the drawings. The dimensions of each portion in the drawings used in the following explanations are not limited to being identical to the actual dimensions, and can be appropriately modified.

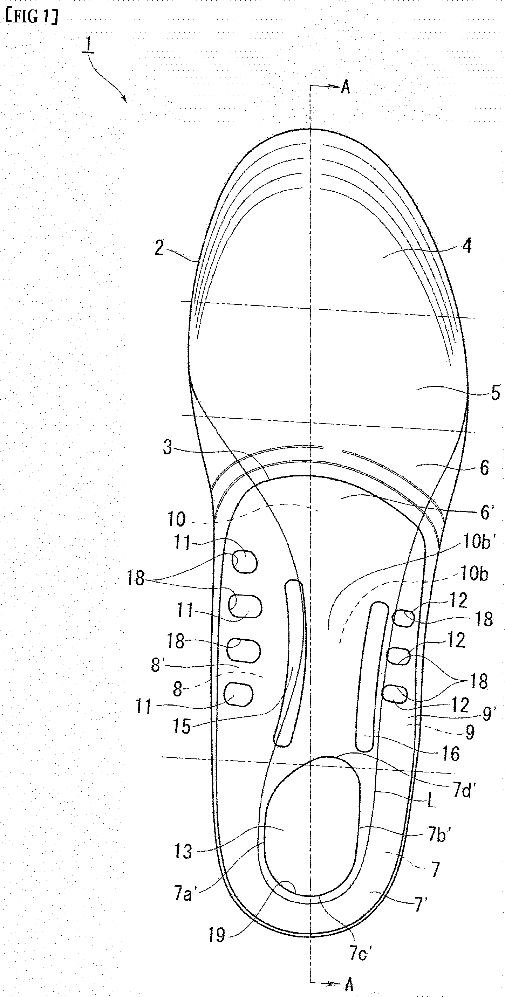

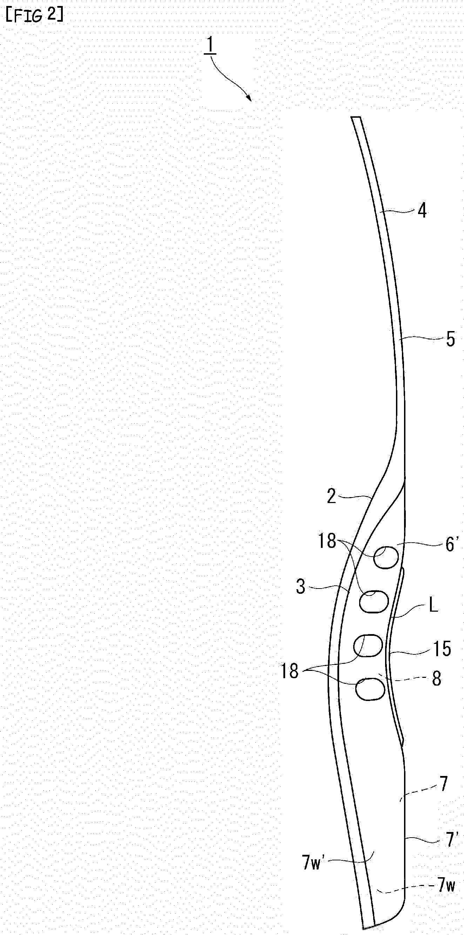

As shown in FIGS. 1 and 2, a shoe insole 1 according to one embodiment of the present invention includes a sheet section 2 disposed on the entire base of a shoe (not illustrated), and a support plate 3 that covers the sheet section 2 from below over an area from a central portion to a heel portion in the longitudinal direction of the sheet section 2.

The sheet section 2 includes, from the distal end to the rear end, a toe section 4, a bulged section 5 at which the left-right width is most bulged, an arch section 6, and a heel section 7.

The toe section 4 is a portion which is formed such that the toes of the foot are disposed therein.

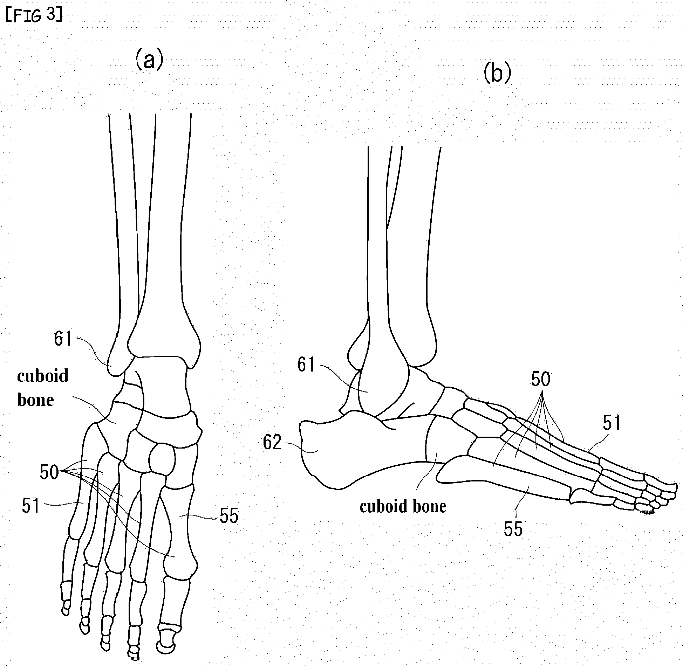

The bulged section 5 is a portion which is formed such that the tips of the metatarsal bones 50 as shown in FIG. 3 (i.e. from the thenar eminence to the hypothenar eminence), at which the width of the foot protrudes farthest to the left and right, are disposed therein.

The heel section 7 is a portion which is formed such that a section of the foot directly below the lateral malleolus 61 to the heel bone 62 rearward of the lateral malleolus 61 as illustrated in FIG. 3 is disposed therein.

The arch section 6 is a portion which is formed such that a section of the foot from the metatarsal bones 50 (excluding the bulged section 5) to directly below the lateral malleolus 61 as illustrated in FIG. 3 is disposed therein.

The outer shape of the sheet section 2 is formed so as to follow the approximate outer shape of the overall foot in a plan view.

The arch section 6 of the sheet section 2 includes: an inner wall section 8 formed such that mainly a first metatarsal bone 51 shown in FIG. 3 is disposed therein; an outer wall section 9 formed such that mainly a fifth metatarsal bone 55 shown in FIG. 3 is disposed therein; and a central wall section 10 formed between the inner wall section 8 and the outer wall section 9.

In more detail, the inner wall section 8 is a portion that is formed to be curved in an arch shape which protrudes upward with an end section standing smoothly upward such that mainly the first metatarsal bone 51 shown in FIG. 3 is disposed therein. A plurality of protrusions 11 are formed spaced apart by intervals in the longitudinal direction on the bottom surface side of the inner wall section 8.

The outer wall section 9 is a portion that is formed to be curved in an arch shape which protrudes upward with an end section standing upward such that mainly the fifth metatarsal bone 55 shown in FIG. 3 is disposed therein. A plurality of protrusions 12 are formed spaced apart by intervals in the longitudinal direction on the bottom surface side of the outer wall section 9.

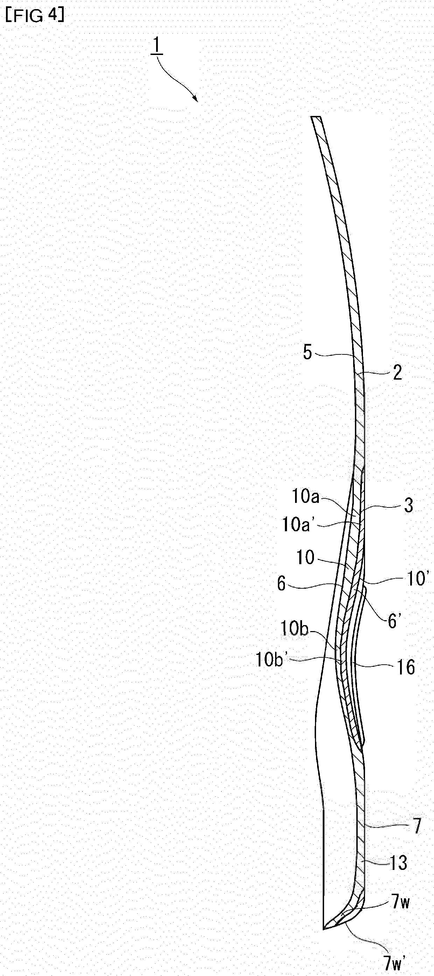

As shown in FIG. 4, the central wall section 10 has, in the cross-section view along A-A of FIG. 1, a central front wall section 10a in which both the upper and bottom surfaces are inclined upward in an approximately linear shape toward the toe section 4, and a central rear wall section 10b in which both the upper and bottom surfaces are curved so as to draw an arch shape in the front-rear direction.

The central rear wall section 10b is formed with an approximately fixed thickness, and the central front wall section 10a is formed to be slightly thicker than the central rear wall section 10b.

As shown in FIGS. 2 and 4, the heel section 7 of the sheet section 2 has, on an end section thereof, a standing up section 7w so that a lower end portion of the heel of the foot can be surrounded and held from a side surface side. The inner wall section 8, the standing up section 7w, and the outer wall section 9 are smoothly continuous.

As shown in FIGS. 1 and 4, a thick section 13 which absorbs the body weight applied to the heel section 7 is formed on a bottom surface side of the heel section 7 of the sheet section 2.

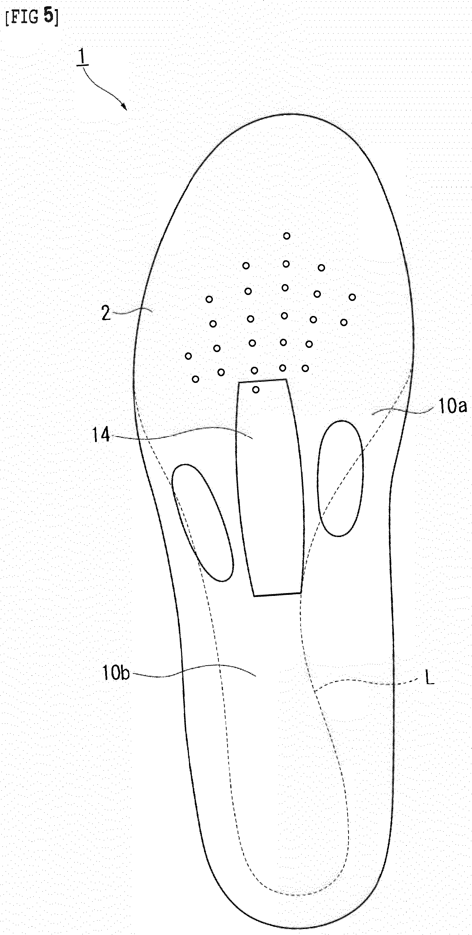

As shown in FIG. 5, a central convex section 14 which is raised and extends in the front-rear direction is formed on the upper surface of the sheet section 2 approximately in the center in the width direction of the central front wall section 10a. The central convex section 14 is formed so as to be able to fit into a recess of the inner foot immediately to the rear of the thenar eminence that supports the body weight when standing on so-called tiptoe. The left and right sides of the central convex section 14 are relatively slightly recessed.

The sheet section 2 is molded from rubber or a synthetic resin, etc., and is preferably formed a soft synthetic resin such as ethylene-vinyl acetate copolymer (EVA). The sheet section 2 should be formed to have a degree of hardness in the range of 22 to 80 as measured using an Asker rubber durometer (C-type).

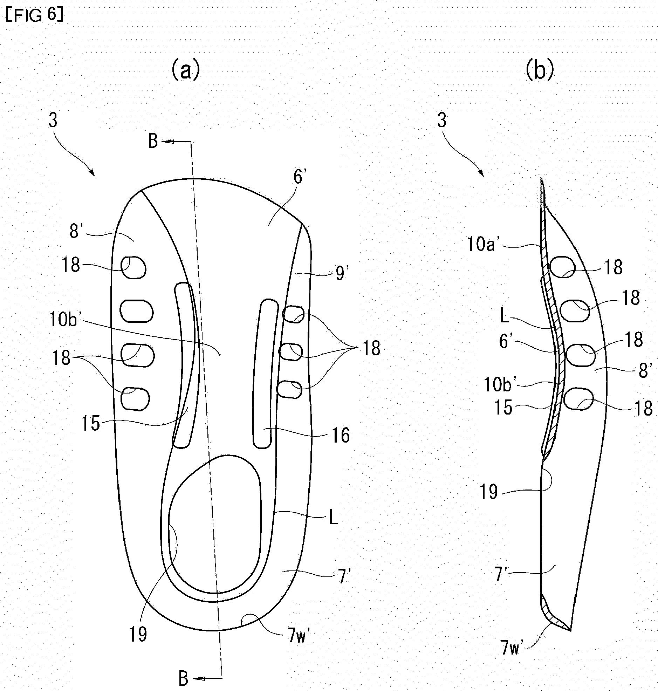

As shown in FIG. 1, 4, or 6, the support plate 3 closely adheres along the shape of the sheet section 2 so as to cover from the arch section 6 to the heel section 7.

Specifically, the support plate 3 includes the following sections which correspond to the sections of the sheet section 2: an arch section 6'; an inner wall section 8', an outer wall section 9', a central front wall section 10a', and a central rear wall section 10b' that constitute the arch section 6'; and a heel section 7'. The heel section 7' of the support plate 3 also has a standing up section 7w' that surrounds and holds the side surfaces of the heel of the foot.

The support plate 3 has an inner ridge section 15 and an outer ridge section 16 formed respectively on approximately the boundary between the inner wall section 8' and the central rear wall section 10b' and on approximately the boundary between the outer wall section 9' and the central rear wall section 10b', i.e. approximately on or along a line L.

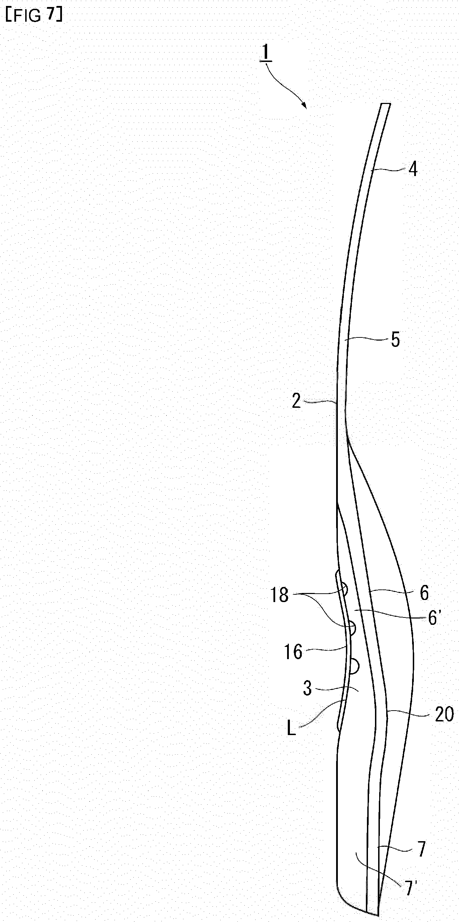

As shown in FIG. 2, the inner ridge section 15 is formed in an arch shape approximately parallel to the arch shape appearing at the lower end when viewing the inner wall section 8' from the side surface. Further, as shown in FIG. 7, the outer ridge section 16 is formed in an arch shape approximately parallel to the arch shape appearing at the lower end when viewing the outer wall section 9' from the side surface.

The inner ridge section 15 and the outer ridge section 16 are both formed in a band shape with an approximately fixed width dimension.

The central front wall section 10a' of the support plate 3 is formed in the same shape as the sheet section 2, i.e. an approximately flat shape. The central rear wall section 10b' of the support plate 3 has an approximately fixed thickness overall, and the upper surface and bottom surface thereof form a longitudinal direction arch shape nearly identical to the bottom surface of the central rear wall section 10b of the sheet section 2 and the inner ridge section 15 and the outer ridge section 16.

As shown in FIG. 1, in the standing up section of the inner wall section 8', a plurality of through-holes 18 are formed spaced apart along the longitudinal direction of the support plate 3. The through-holes 18 formed in the inner wall section 8' of the support plate 3 are formed so that the protrusions 11 formed on the bottom surface side of the inner wall section 8 of the sheet section 2 can fit therein without any gaps. The bottom surface of the support plate 3 and the top surface of the protrusions 11 are formed so as to be flush with each other.

In the outer wall section 9', a plurality of the through-holes 18 are formed spaced apart along the longitudinal direction of the support plate 3. The through-holes 18 formed in the outer wall section 9' of the support plate 3 are formed so that the protrusions 12 formed on the bottom surface side of the outer wall section 9 of the sheet section 2 can fit therein without any gaps. The bottom surface of the support plate 3 and the top surface of the protrusions 12 are formed so as to be flush with each other.

In this way, the support plate 3 is formed to match the shape of each section of the sheet section 2, and thereby, as shown in FIGS. 1 and 5, the inside of the line L serving as the boundary generally contacts a horizontal plane such as the surface of the ground except for the central rear wall section 10b', and the outside of the line L is formed so as to stand up smoothly with the line L as a boundary. Further, the arch section 6 is firmly raised up by the central rear wall section 10b' and the inner ridge section 15 and the outer ridge section 16 which are formed approximately on the line L, and thus the longitudinal arch of the foot can be supported.

In the heel section 7' of the support plate 3, an opening 19 which absorbs and receives a load applied from the heel is formed.

The opening 19 is formed approximately parallel to the line L on an inner side 7a' and an outer side 7b' of the heel section 7', and extends slightly inward so as to separate from the line L on a rear side 7c' of the heel section 7'. Further, on the outside of a distal end side 7d', the opening 19 expands approaching the central rear wall section 10b', which gradually warps up diagonally.

Due to this configuration, it can be anticipated that a load which extends between the heel of the foot and the outside in the width direction of the arch of the foot and which may be applied to the outer tendons and the like, onto which the body weight is prone to be strongly loaded, can be reduced, and the body weight which is applied approximately vertically downward from the lateral malleolus 61 can be softly received.

In particular, if the thickness of the sheet section 2 is formed to be thin within an allowable range so as to attempt to form the shoe insole 1 to be slim, the support plate 3 fits closer to the sole of the foot, and thus the portions at which the body weight is prone to be loaded, such as the heel and tendons, etc. of the foot, more readily receive the load caused by the hardness of the support plate 3. However, by forming the opening 19 with the shape described above, it can be anticipated that the load on the heels and tendons, on which a large body weight is prone to be loaded, can be effectively reduced, and the fit of the sheet section 2 and the support plate 3 can be highly maintained.

As shown in FIG. 4, the thickness of the support plate 3 is formed to be a fixed thickness, excluding the edges which surround the central front wall section 10a, the through-holes 18, and the opening 19 as well as the edge that serves as the outer peripheral edge of the support plate 3. Specifically, the thickness of the support plate 3 is formed to be 1.1 mm to less than 2.1 mm, preferably about 2 mm.

The central front wall section 10a' of the support plate 3 is formed to be about 10% thinner than the central rear wall section 10b' and the heel section 7', etc. Specifically, the central front wall section 10a' is formed with a thickness of 1.0 mm to less than 1.9 mm.

The outer peripheral edge of the support plate 3 and the edges that form the through-holes 18 and the opening 19 are chamfered so as to fit easily to the sheet section 2.

The inner ridge section 15 and the outer ridge section 16 of the support plate 3 protrude by 0.9 mm to less than 1.1 mm, preferably 1.0 mm, from the top surface of the central rear wall section 10b' which surrounds them. The inner ridge section 15 and the outer ridge section 16 are both formed to be solid.

The support plate 3 is formed by a hard resin such as a thermoplastic polyurethane (TPU). The support plate 3 is formed with a degree of hardness of from 40D to less than 72D, or from 45D to less than 65D, as measured using an Asker rubber durometer (D-type).

The entirety of the upper surface of the support plate 3 is closely fitted to the bottom surface of the sheet section 2 by compression bonding so that there are no gaps therebetween and the support plate 3 cannot be easily separated from the sheet section 2. The protrusions 11 and 12 and the thick section 13 formed on the sheet section 2 are fitted into the corresponding through-holes 18 and the opening 19 of the support plate 3, and thus are approximately flush with the top surface of the support plate 3.

The shoe insole 1 of the present invention having the configuration described above achieves the following effects.

The elastic force of the longitudinal arch of the shoe insole 1 is further increased due to the longitudinal arch shapes having approximately the same curvature of the sheet section 2, the lower end of the inner wall section 8' and the lower end of the outer wall section 9' of the support plate 3, the inner ridge section 15, the outer ridge section 16, and the top and bottom surfaces of the central rear wall section 10b'. Further, due to the arch shapes of the inner ridge section 15, the outer ridge section 16, and the central rear wall section 10b', the central portion in the width direction of the arch of the foot (between the inner longitudinal arch and the outer longitudinal arch) can be flexibly and reliably held in a longitudinal arch shape.

A longitudinal arch shape is firmly formed in the top and bottom surfaces of the central rear wall section 10b of the sheet section 2, and the inner ridge section 15, the outer ridge section 16, and the upper surface of the central rear wall section 10b' of the support plate 3 securely retain this longitudinal arch shape. Therefore, any unevenness in the body weight of the thenar eminence and the hypothenar eminence placed on the bulged section 5 and the body weight of the heel placed on the heel section 7 can be prevented. Accordingly, the center of gravity can be guided to the correct position by the shape of the shoe insole 1.

In addition, the bones and joints of the foot can be appropriately held without forming any unnecessary gaps on the sole of the foot by the aforesaid sections as well as the central convex section 14 formed on the central front wall section 10a of the sheet section 2.

The first metatarsal bone 51 and the fifth metatarsal bone 55 shown in FIG. 3 as well as the lower side surface of the heel can be firmly, but without becoming cramped, held by the inner wall section 8', the outer wall section 9', and the standing up section 7w' of the heel section 7'.

By reciprocally combining the above-described effects, the bones of the foot which are connected by a plurality of joints can be prevented from deviating or distorting, and can be easily retained in an appropriately assembled state. Thus, an effect is achieved in which distortions in the posture based on a deviation in the bones of the foot can be prevented during exercise such as walking.

Meanwhile, there was a problem with the support plate 3 of the shoe insole 1 of the present invention in that slight pain developed in the foot after use over a long period of time even when the support plate 3 was within the range of hardness of a TPU resin which is generally used in other shoe insoles. This was believed to be likely based on the shape of the support plate 3 of the present invention, but as a result of earnest research focused on this problem, the problem was eliminated by setting the degree of hardness of the support plate 3 to from 40D to 72D.

As shown in FIG. 7, the sheet section 2 and the support plate 3 preferably include an extension section 20 that protrudes upward so as to oppose, from a side surface, the heel sections 7, 7' located directly below the lateral malleolus 61 shown in FIG. 3 and/or the base end section of the arch sections 6, 6'. In this case, the extension sections 20 should be approximately parallel in both the sheet section 2 and the support plate 3, i.e. the peripheral edge of the sheet section 2 and the peripheral edge of the support plate 3 are approximately parallel in the extension section 20.

Due to the above configuration, an effect is achieved in which supination of the foot (in other words, a movement in which the foot rolls around the little toe side) can be prevented.

The sheet section 2 may include two foamed resin layers having different degrees of hardness and impact resilience coefficients. A first layer on an upper surface side and a second layer provided below the first layer preferably constitute impact resilient resin layers comprising, as a main component, an ethylene-vinyl acetate copolymer.

Further, the measured degrees of hardness as measured using an Asker rubber durometer (C-type) and the impact resilience coefficients as measured according to ISO4662:2009 of the first layer and the second layer preferably satisfy the following relationships.
Measured Degree of Hardness: the first layer < the second layer, and
Impact Resilience Coefficient: the first layer < the second layer

Specifically, as the first layer, a resin comprising EVA as a main component is used, and in order to adjust the impact resilience coefficient, the first layer can be configured as a resin layer into which impact absorbing materials such as polyethylene, polypropylene, and a silicon resin are mixed.

More specifically, a resin having a degree of hardness as measured using an Asker rubber durometer (C-type) of 23 to 28, and an impact resilience coefficient as measured according to ISO4662:2009 of 47% to 53%, more preferably 49% to 52%, can be suitably used.

In addition, the first layer may be a polyurethane foamed resin layer.

As the second layer, specifically, a resin comprising EVA as a main component is used, and in order to adjust the impact resilience coefficient, a resin obtained by compressing a foamed resin comprising EVA as a main component can be used. More specifically, the degree of hardness as measured using an Asker rubber durometer (C-type) should be 50 to 60, and the impact resilience coefficient as measured according to ISO4662:2009 should be 55% to 65%. Further, the degree of hardness as measured using an Asker rubber durometer (C-type) is preferably 53 to 60, and the impact resilience coefficient as measured according to ISO4662:2009 is preferably 57% to 59%.

The first layer and the second layer are pressed, and then a sheet layer made of fiber is provided on the top surface of the first layer. In this state, although not limited thereto, the first layer is formed with a thickness of from 0.5 mm to 4.0 mm. Further, although not limited thereto, the second layer is formed with a thickness of from 1.0 mm to 4.0 mm. The second layer may be formed to be thicker at the heel than at the toe, and if the first layer is a polyurethane layer, the first layer and the second layer may both formed to be thicker than in the case that the first layer and the second layer comprise EVA as a main component.

Due to the above configuration, the second layer is imparted with an appropriate degree of hardness and resilience, and thereby it exerts a cushioning property on the foot. In addition, the first layer is imparted with a low degree of hardness, i.e. flexibility, and low resilience, and thereby the first layer can be firmly retained following the shape of the sole of the foot, and the foot can be softly supported. Accordingly, an effect is achieved in which the skeletal structure of the foot can be held as correctly as possible, and the foot can be effectively protected from impacts thereto.

### EXAMPLES

In the following, the present invention shall be explained in detail using examples, but the scope of the present invention should not be construed as limited to these examples.

### [EXAMPLE 1]

A shoe insole as indicated in the above embodiments was produced, comprising the sheet section 2 made of a soft synthetic resin such as ethylene-vinyl acetate copolymer (EVA) and having a degree of hardness as measured using an Asker rubber durometer (C-type) of about 55, and the support plate 3 made of a hard resin such as a thermoplastic polyurethane (TPU) and having a degree of hardness of 40D as measured using an Asker rubber durometer (D-type).

### [EXAMPLE 2]

A shoe insole identical to that of Example 1 was produced, except that the degree of hardness of the support plate 3 was 55D as measured using an Asker rubber durometer (D-type).

### [EXAMPLE 3]

A shoe insole identical to that of Example 1 was produced, except that the degree of hardness of the support plate 3 was 63.5D as measured using an Asker rubber durometer (D-type).

### [COMPARATIVE EXAMPLE]

A shoe insole identical to that of Example 1 was produced, except that the degree of hardness of the support plate 3 was 72D (comparative example) as measured using an Asker rubber durometer (D-type).

### (Evaluation Method)

An adult male A having a height of about 172 cm and a body weight of 75 kg, an adult male B having a height of about 162 cm and a body weight of 53 kg, an adult female C having a height of about 160 cm and a body weight of 52 kg, and an adult female D having a height of about 164 cm and a body weight of 56 kg were made to wear exercise shoes in which the shoe insoles of Examples 1 to 3 and the shoe insole of the comparative example were disposed. The feeling of use was evaluated upon walking on asphalt for about 2 hours. The feeling of use was evaluated regarding (1) the presence/absence of a feeling of support or an uncomfortable feeling in the sole of the foot, (2) the presence/absence of the occurrence of pain in the sole of the foot, and (3) the presence/absence of the occurrence of pain anywhere besides the sole of the foot when walking for no more than 2 hours. In these examples, a "feeling of support" indicates whether there is a sensation that the sole of the foot is being held (retained) by the insole, and an "uncomfortable feeling" indicates localized or overall discomfort. The evaluation results are shown below in Tables 1 to 4.

**[TABLE 1]**

| **EXAMPLE 1** | **Male A** | **Male B** | **Female C** | **Female D** |
|---|---|---|---|---|
| (1) Presence/absence of feeling of support or uncomfortable feeling in sole | No uncomfortable feeling. Feeling of support slightly weak. | No uncomfortable feeling. | No uncomfortable feeling. Feeling of support felt somewhat weak in heel and arch of foot. | No uncomfortable feeling. Feeling of support slightly weak. |
| (2) Presence/absence of pain in sole | Slight fatigue in overall sole of foot. | None in particular. | Slight feeling of pain in root of middle toe and fourth toe on one foot. | None in particular. |
| (3) Presence/absence of pain anywhere besides sole | None in particular. | Felt fatigue in thigh and calf. | None in particular. | None in particular. |

**[TABLE 2]**

| **EXAMPLE 2** | **Male A** | **Male B** | **Female C** | **Female D** |
|---|---|---|---|---|
| (1) Presence/absence of feeling of support or uncomfortable feeling in sole | Felt appropriate amount of support. | Felt appropriate amount of support. | Felt appropriate amount of support. | Felt appropriate amount of support. |
| | No uncomfortable feeling. | No uncomfortable feeling. | No uncomfortable feeling. | No uncomfortable feeling. |
| (2) Presence/absence of pain in sole | Experienced no fatigue at about 2 hours. | Experienced no fatigue at about 2 hours. | Experienced no fatigue at about 2 hours. | Experienced no fatigue at about 2 hours. |
| (3) Presence/absence of pain anywhere besides sole | Experienced no fatigue at about 2 hours. | Experienced no fatigue at about 2 hours. | Experienced no fatigue at about 2 hours. | Experienced no fatigue at about 2 hours. |

**[TABLE 3]**

| **EXAMPLE 3** | **Male A** | **Male B** | **Female C** | **Female D** |
|---|---|---|---|---|
| (1) Presence/absence of feeling of support or uncomfortable feeling in sole | Felt slightly uncomfortable in heel and forward inner side. | No uncomfortable feeling in particular. | Felt like heel was being held immediately upon wearing shoe, and felt a close fit over entire sole. | Felt slightly uncomfortable in arch of foot. |
| (2) Presence/absence of pain in sole | Was able to use without any problems for around 2 hours, but started to feel slight pain once a long period (over 2 hours) had elapsed. | Felt pain due to chafing at edge portion of heel cup. | None in particular. | Experienced a very slight sensation of pain and uncomfortable feeling. |
| (3) Presence/absence of pain anywhere besides sole | Experienced no fatigue at about 2 hours. | None in particular. | None in particular. | None in particular. |

**[TABLE 4]**

| **COMP. EX.** | **Male A** | **Male B** | **Female C** | **Female D** |
|---|---|---|---|---|
| (1) Presence/absence of feeling of support or uncomfortable feeling in sole | Felt uncomfortable on front inner side of heel bone. | Felt uncomfortable in sustentaculum tali. | Experienced a feeling of a foreign object being present at location near space between arch of foot and heel. | Felt uncomfortable immediately upon wearing shoe. Arch felt stiff. |
| | | Felt pressure on sole of foot. | | |
| (2) Presence/absence of pain in sole | Felt uncomfortable at front inner side of heel bone at about 1 hour of use. Felt pain at same location soon after. | Upon walking, felt pain due to chafing at edge portion of heel cup. | Felt pain after about 15 minutes, and this pain continued for about 25 minutes thereafter. | Felt slight numbness after about 10 minutes. Felt pain after about 30 minutes, and could not walk naturally. |
| (3) Presence/absence of pain anywhere besides sole | Experienced no fatigue at about 2 hours. | Experienced no fatigue at about 2 hours. | None in particular. | None in particular. |

In the evaluation results shown in Tables 1 to 4, the shoe insole in which the support plate 3 had a degree of hardness of 40D did not cause an uncomfortable feeling, but the feeling of support was slightly weak.

The shoe insole in which the support plate 3 had a degree of hardness of 55D exhibited a good feeling of support without any uncomfortable feelings, and did not readily cause pain in the sole of the foot. Further, since this shoe insole also did not readily cause pain in other areas besides the sole of the foot, it can be surmised that the shape of the sole of the foot was satisfactorily retained.

The shoe insole in which the support plate 3 had a degree of hardness of 63.5D provided a feeling of support, but also caused slightly uncomfortable feelings, and caused pain in the sole of the foot after a prolonged period of use.

The shoe insole in which the support plate 3 had a degree of hardness of 72D was prone to cause uncomfortable feelings, and caused pain in the sole of the foot even after use over a short period.

### REFERENCE SIGNS LIST

1: shoe insole
2: sheet section
3: support plate
4: toe section
5: bulged section
6: arch section
7: heel section
8: inner wall section
9: outer wall section
10: central wall section
10a: central front wall section
10b: central rear wall section
13: thick section (portion of sheet section)
14: central convex section
15: inner ridge section
16: outer ridge section
18: through-hole
19: opening

## Claims

1. A shoe insole (1) comprising:
a sheet section (2) disposed on an entire base of a shoe, the sheet section (2) including a toe section (4) formed such that the toes of a foot can be disposed therein, a bulged section (5) formed such that tips of metatarsal bones (50) at which the width of the foot protrudes farthest to the left and right can be disposed therein, an arch section (6) formed such that a section of the foot from the metatarsal bones (50) to directly below the lateral malleolus (61) can be disposed therein, and a heel section (7) formed such that a section of the foot from directly below the lateral malleolus (61) to the heel bone (62) rearward of the lateral malleolus (61) can be disposed therein; and
a support plate (3) that is adhered to a bottom surface of the arch section (6) and the heel section (7) of the sheet section (2), and that is formed from a material which is harder than the sheet section (2),
wherein the sheet section (2) and the support plate (3) each comprises in the arch section (6): an inner wall section (8, 8') curved in an arch shape with an end section standing upward such that at least a first metatarsal bone (51) is disposed therein; an outer wall section (9, 9') curved in an arch shape with an end section standing upward such that at least a fifth metatarsal bone (55) is disposed therein; a central wall section (10, 10') formed between the inner wall section (8, 8') and the outer wall section (9, 9'),
the support plate (3) comprises an inner ridge section (15) formed on a boundary between the inner wall section (8') and the central wall section (10'), and an outer ridge section (16) formed on a boundary between the outer wall section (9') and the central wall section (10'),
a plurality of through-holes (18), arranged in the longitudinal direction of the support plate (3), are formed in the inner wall section (8') and the outer wall section (9') of the support plate (3),
**characterized in that** at least an upper surface of the central wall section (10') located between the inner ridge section (15) and the outer ridge section (16) is curved so as to draw an arch in the longitudinal direction of the support plate (3), and
an upper surface and a bottom surface of a central rear wall section (10b') of the support plate (3), located between the inner ridge section (15) and the outer ridge section (16), form a longitudinal direction arch shape which is nearly identical to a bottom surface of a central rear wall section (10b) of the sheet section (2) and the inner ridge section (15) and the outer ridge section (16).

2. The shoe insole (1) according to claim 1, wherein the sheet section (2) and the support plate (3) each comprises an extension section (20) that protrudes upward so as to oppose, from a side surface, the heel section (7) located directly below the lateral malleolus (61) and/or a base end section of the arch section (6).

3. The shoe insole (1) according to claim 1 or 2, wherein the support plate (3) is formed with a degree of hardness of from 40D to less than 72D, and is arranged along a bottom surface shape of the sheet section (2) with a fixed thickness.

4. The shoe insole (1) according to claim 1 or 2, wherein the support plate (3) is formed with a degree of hardness of from 40D to less than 65D, and is arranged along a bottom surface shape of the sheet section (2) with a fixed thickness.

5. The shoe insole (1) according to any one of claims 1 to 4, wherein in the heel section (7) of the support plate (3), an opening (19) into which a portion of the sheet section (2) is fitted is formed.

6. The shoe insole (1) according to any one of claims 1 to 5, wherein the sheet section (2) comprises at least two foamed resin layers having different degrees of hardness and impact resilience coefficients, wherein a first layer on an upper surface side and a second layer provided below the first layer constitute impact resilient resin layers comprising, as a main component, an ethylene-vinyl acetate copolymer,
wherein a measured degree of hardness as measured using an Asker rubber durometer C-type and an impact resilience coefficient as measured according to ISO4662 of the first layer and the second layer satisfy the following relationships:
Measured Degree of Hardness: the first layer < the second layer
Impact Resilience Coefficient: the first layer < the second layer.

7. The shoe insole (1) according to claim 6, wherein the measured degree of hardness as measured using an Asker rubber durometer C-type of the second layer is 50 to 60, and the impact resilience coefficient as measured according to ISO4662 of the second layer is 55% to 65%.

8. The shoe insole (1) according to claim 6 or 7, wherein the measured degree of hardness as measured using an Asker rubber durometer C-type of the first layer is 23 to 28, and the impact resilience coefficient as measured according to ISO4662 of the first layer is 47% to 53%.

9. The shoe insole (1) according to any one of claims 1 to 5, wherein the sheet section (2) comprises at least two foamed resin layers having different impact resilience coefficients,
wherein a first layer on an upper surface side constitutes a polyurethane foamed resin layer,
wherein a second layer provided below the first layer constitutes an impact resilient resin layer comprising, as a main component, an ethylene-vinyl acetate copolymer, and
wherein the measured degree of hardness as measured using an Asker rubber durometer C-type of the second layer is 50 to 60, and the impact resilience coefficient as measured according to ISO4662 of the second layer is 55% to 65%.

## Patentansprüche

1. Schuheinlage (1), umfassend:
einen flächigen Bereich (2), der auf einer gesamten Basis eines Schuhs angeordnet ist, wobei der flächige Bereich (2) einen Zehenbereich (4), der derart ausgebildet ist, dass die Zehen eines Fußes darin angeordnet werden können, einen gewölbten Bereich (5), der ausgebildet ist, sodass Spitzen von Mittelfußknochen (50), an denen die Breite des Fußes am weitesten nach links und rechts vorsteht, darin angeordnet werden können, einen Bogenbereich (6), der ausgebildet ist, sodass ein Bereich des Fußes von den Mittelfußknochen (50) bis unmittelbar unterhalb des Außenknöchels (61) darin angeordnet werden kann, und einen Fersenbereich (7) beinhaltet, der ausgebildet ist, sodass ein Bereich des Fußes von unmittelbar unterhalb des Außenknöchels (61) bis zu dem Fersenbein (62) hinter dem Außenknöchel (61) darin angeordnet werden kann; und
eine Stützplatte (3), die an einer unteren Oberfläche des Bogenbereichs (6) und des Fersenbereichs (7) des flächigen Bereichs (2) geklebt ist und aus einem Material ausgebildet ist, das härter ist als der flächige Bereich (2),
wobei der flächige Bereich (2) und die Stützplatte (3) in dem Bogenbereich (6) jeweils umfassen: einen Innenwandbereich (8, 8'), der in einer Bogenform gekrümmt ist, wobei ein Endbereich nach oben steht, sodass wenigstens ein erster Mittelfußknochen (51) darin angeordnet ist; einen Außenwandbereich (9, 9'), der in einer Bogenform gekrümmt ist, wobei ein Endbereich nach oben steht, sodass wenigstens ein fünfter Mittelfußknochen (55) darin angeordnet ist; einen mittleren Wandbereich (10, 10'), der zwischen dem Innenwandbereich (8, 8') und dem Außenwandbereich (9, 9') ausgebildet ist,
die Stützplatte (3) einen Innenwulstbereich (15) umfasst, der an einer Grenze zwischen dem Innenwandabschnitt (8') und dem mittleren Wandabschnitt (10') ausgebildet ist, und einen Außenwulstbereich (16), der an einer Grenze zwischen dem Außenwandbereich (9') und dem mittleren Wandbereich (10') ausgebildet ist,
eine Mehrzahl von Durchgangslöchern (18), die in der Längsrichtung der Stützplatte (3) angeordnet sind, in dem Innenwandbereich (8') und dem Außenwandbereich (9') der Stützplatte (3) ausgebildet sind,
**dadurch gekennzeichnet, dass** wenigstens eine obere Oberfläche des mittleren Wandbereichs (10'), die sich zwischen dem Innenwulstbereich (15) und dem Außenstegbereich (16) befindet, gekrümmt ist, um einen Bogen in der Längsrichtung der Stützplatte (3) zu zeichnen, und
eine obere Oberfläche und eine untere Oberfläche eines mittleren Rückwandbereichs (10b') der Stützplatte (3), die sich zwischen dem Innenwulstbereich (15) und dem Außenwulstbereich (16) befinden, eine Bogenform in Längsrichtung ausbilden, die mit der unteren Oberfläche eines mittleren Rückwandabschnitts (10b) des flächigen Bereichs (2) und dem Innenwulstbereich (15) und dem Außenstegbereich (16) nahezu identisch ist.

2. Schuheinlage (1) nach Anspruch 1, wobei der flächige Bereich (2) und die Stützplatte (3) jeweils einen Erweiterungsbereich (20) umfassen, der nach oben vorsteht, um dem Fersenbereich (7), der sich unmittelbar unter dem Außenknöchel (61) und/oder einem Basisendbereich des Bogenbereichs (6) befindet, von einer Seitenoberfläche gegenüberzustehen.

3. Schuheinlage (1) nach Anspruch 1 oder 2, wobei die Stützplatte (3) mit einem Härtegrad von 40D bis weniger als 72D ausgebildet ist, und entlang einer unteren Oberflächenform des flächigen Bereichs (2) mit einer festen Dicke angeordnet ist.

4. Schuheinlage (1) nach Anspruch 1 oder 2, wobei die Stützplatte (3) mit einem Härtegrad von 40D bis weniger als 65D ausgebildet ist und entlang einer unteren Oberfläche des flächigen Bereichs (2) mit einer festen Dicke angeordnet ist.

5. Schuheinlage (1) nach einem der Ansprüche 1 bis 4, wobei in dem Fersenbereich (7) der Stützplatte (3) eine Öffnung (19) ausgebildet ist, in die ein Abschnitt des flächigen Bereichs (2) eingepasst wird.

6. Schuheinlage (1) nach einem der Ansprüche 1 bis 5, wobei der flächige Bereich (2) wenigstens zwei geschäumte Harzschichten mit unterschiedlichen Härte- und Schlagzähigkeitskoeffizienten umfasst, wobei eine erste Schicht auf einer oberen Oberflächenseite und eine zweite Schicht, die unterhalb der ersten Schicht bereitgestellt ist, schlagzähe Harzschichten bilden, die als eine Hauptkomponente ein Ethylen-Vinylacetat-Copolymer umfassen,
wobei ein gemessener Härtegrad, gemessen unter Verwendung eines Asker-Kautschuk-Durometers Typ C, und ein Schlagzähigkeitskoeffizient, gemessen gemäß ISO4662, der ersten Schicht und der zweiten Schicht die folgenden Beziehungen erfüllen:
Gemessener Härtegrad: die erste Schicht < die zweite Schicht
Schlagzähigkeitskoeffizient: die erste Schicht < die zweite Schicht.

7. Schuheinlage (1) nach Anspruch 6, wobei der gemessene Härtegrad, gemessen unter Verwendung eines Asker-Kautschuk-Durometers Typ C, der zweiten Schicht 50 bis 60 beträgt und der Schlagzähigkeitskoeffizient der zweiten Schicht, gemessen gemäß ISO4662, 55 % bis 65 % beträgt.

8. Schuheinlage (1) nach Anspruch 6 oder 7, wobei der gemessene Härtegrad, gemessen unter Verwendung eines Asker-Kautschuk-Durometers Typ C, der ersten Schicht 23 bis 28 beträgt und der Schlagzähigkeitskoeffizient der ersten Schicht, gemessen gemäß ISO4662, 47 % bis 53 % beträgt.

9. Schuheinlage (1) nach einem der Ansprüche 1 bis 5, wobei der flächige Bereich (2) wenigstens zwei geschäumte Harzschichten mit unterschiedlichen Schlagzähigkeitskoeffizienten umfasst,
wobei eine erste Schicht auf einer oberen Oberflächenseite eine aus Polyurethan geschäumte Harzschicht bildet,
wobei eine zweite Schicht, die unterhalb der ersten Schicht bereitgestellt ist, eine schlagzähe Harzschicht bildet, die als eine Hauptkomponente ein Ethylen-Vinylacetat-Copolymer umfasst, und
wobei der gemessene Härtegrad, gemessen unter Verwendung eines Asker-Kautschuk-Durometers Typ C, der zweiten Schicht 50 bis 60 beträgt und der Schlagzähigkeitskoeffizient der zweiten Schicht, gemessen gemäß ISO4662, 55 % bis 65 % beträgt.

## Revendications

1. Semelle intérieure de chaussure (1) comprenant :
une section feuille (2) disposée sur une base toute entière d'une chaussure, cette section feuille (2) comprenant une section orteils (4) formée de manière à ce que les orteils d'un pied puissent être disposés à l'intérieur de celle-ci, une section bombée (5) formée de manière à ce que les pointes des os métatarsiens (50) auxquelles la largeur du pied fait saillie le plus loin sur la gauche et sur la droite puissent être disposées à l'intérieur de celle-ci, une section arche (6) formée de manière à ce que qu'une section du pied allant des os métatarsiens (50) jusqu'à juste en dessous de la malléole latérale (61) puisse être disposée à l'intérieur de celle-ci, et une section talon (7) formée de manière à ce qu'une section du pied depuis juste en dessous de la malléole latérale (61) jusqu'au calcanéum (62) vers l'arrière de la malléole latérale (61) puisse être disposée à l'intérieur de celle-ci ; et
une plaque de support (3) qui est collée à une surface inférieure de la section arche (6) et de la section talon (7) de la section feuille (2) et qui est formée à partir d'une matière qui est plus dure que la section feuille (2),
la section feuille (2) et la plaque de support (3) comportant chacune dans la section arche (6) : une section paroi intérieure (8, 8') incurvée dans la forme d'une arche avec une section extrême orientée vers le haut de manière à ce que qu'au moins un premier os métatarsien (51) soit disposé à l'intérieur de celle-ci ; une section paroi extérieure (9, 9') incurvée dans la forme d'une arche avec une section extrême orientée vers le haut de manière à ce qu'au moins un cinquième os métatarsien (55) soit disposé à l'intérieur de celle-ci ; une section paroi centrale (10, 10') formée entre la section paroi intérieure (8, 8') et la paroi extérieure (9, 9'),
la plaque de support (3) comportant une section nervure intérieure (15) formée sur une délimitation entre la section paroi intérieure (8') et la section paroi centrale (10'), et une section nervure extérieure (16) formée sur une délimitation entre la section paroi extérieure (9') et la section paroi centrale (10'),
une pluralité de trous traversants (18), disposés dans la direction longitudinale de la plaque de support (3), étant formés dans la section paroi intérieure (8') et dans la section paroi extérieure (9') de la plaque de support (3),
**caractérisé en ce qu'**au moins une surface supérieure de la section paroi centrale (10') située entre la section nervure intérieure (15) et la section nervure extérieure (16) est incurvée de façon à former une arche dans la direction longitudinale de la plaque de support (3), et
une surface supérieure et une surface inférieure de la section paroi arrière centrale (10b') de la plaque de support (3), situées entre la section nervure intérieure (15) et la section nervure extérieure (16), constituant une forme d'arche dans la direction longitudinale qui est presque identique à une surface inférieure d'une section paroi arrière centrale (10b) de la section feuille (2) et de la section nervure intérieure (15) et de la section nervure extérieure (16).

2. Semelle intérieure de chaussure (1), dans laquelle la section feuille (2) et la plaque de support (3) comportent chacune une section d'extension (20) qui fait saillie vers le haut afin de s'opposer, depuis une surface latérale, à la section talon (7) située juste en dessous de la malléole latérale (61) et/ou à une section extrémité de base de la section arche (6).

3. Semelle intérieure de chaussure (1) selon la revendication lou 2, dans laquelle la plaque de support (3) est formée avec un degré de dureté allant de 40D à moins que 72D, et est disposée le long d'une surface inférieure de la section feuille (2) avec une épaisseur fixe.

4. Semelle intérieure de chaussure (1) selon la revendication lou 2, dans laquelle la plaque de support (3) est formée avec un degré de dureté allant de 40D à moins que 65D, et est disposée le long d'une forme de la surface inférieure de la section feuille (2) avec une épaisseur fixe.

5. Semelle intérieure de chaussure (1) selon l'une quelconque des revendications 1 à 4, dans laquelle, dans la section talon (7) de la plaque de support (3), une ouverture (19) dans laquelle une partie de la section feuille (2) est montée est formée.

6. Semelle intérieure de chaussure (1) selon l'une quelconque des revendications 1 à 5, dans laquelle la section feuille (2) comporte au moins deux couches de résine formée ayant différents degrés de dureté et différents coefficients de résistance aux chocs, une première couche sur un côté surface supérieure et une deuxième couche prévue en dessous de la première couche constituent des couches de résine résistantes aux chocs comprenant, comme composant principal, un copolymère d'acétate de vinyle-éthylène,
dans laquelle un degré mesuré de dureté tel que mesuré en utilisant un type C de duromètre de caoutchouc Asker et un coefficient de résistance aux chocs tel que mesuré conformément à la norme IS04662 de la première couche et de la deuxième couche satisfont les rapports suivants : Degré mesuré de dureté : celui de la première couche < celui de la deuxième couche
Coefficient de résistance aux chocs : celui de la première couche < celui de la deuxième couche.

7. Semelle intérieure de chaussure (1) selon la revendication 6, dans laquelle le degré mesuré de dureté de la deuxième couche, tel que mesuré en utilisant un type C de duromètre de caoutchouc Asker, est de 50 à 60, et le coefficient de résistance aux chocs de la deuxième couche, tel que mesuré conformément à la norme IS04662, est de 55 % à 65 %.

8. Semelle intérieure de chaussure (1) selon la revendication 6 ou 7, dans laquelle le degré mesuré de dureté de la première couche, tel que mesuré en utilisant un type C de duromètre de caoutchouc Asker, est de 23 à 28, et le coefficient de résistance aux chocs de la première couche, tel que mesuré conformément à la norme IS04662, est de 47 % à 53 %

9. Semelle intérieure de chaussure (1) selon l'une quelconque des revendications 1 à 5, dans laquelle la section feuille (2) comporte au moins deux couches de résine expansée ayant différents coefficients de résistance aux chocs,
une première couche sur un côté surface supérieure constituant une couche en résine polyuréthanne expansée,
une deuxième couche prévue en dessous de la première couche constituant une couche de résine résistante aux chocs comportant, comme composant principal, un copolymère d'acétate de vinyle-éthylène, et
le degré mesuré de dureté de la deuxième couche, tel que mesuré en utilisant un type C de duromètre de caoutchouc Asker, étant de 50 à 60, et le coefficient de résistance aux chocs de la deuxième couche, tel que mesuré conformément à la norme IS04662, est de 55 % à 65 %.
